# EUROPEAN PATENT SPECIFICATION

(11) **EP 3 839 974 B1**
(45) Date of publication and mention of the grant of the patent: **18.01.2023**
(21) Application number: 19218925.6
(22) Date of filing: 20.12.2019
(51) Int. Cl.: G16H 50/30, G16H 10/40

(54) **METHOD FOR ANALYSING THE LIKELIHOOD OF DISEASES**
VERFAHREN ZUR ANALYSE DER WAHRSCHEINLICHKEIT VON KRANKHEITEN
PROCÉDÉ D'ANALYSE DE LA PROBABILITÉ DE MALADIES

(43) Date of publication of application: 23.06.2021
(73) Proprietor: Dynamic Code AB, 583 30 Linköping (SE)
(72) Inventor: Selbing, Anders, 589 35 Linköping (SE); Farnebäck, Malin, 584 22 Linköping (SE)
(74) Representative: Ström & Gulliksson AB

(56) References cited:
- US-A1- 2007 178 495
- US-A1- 2008 221 927

## Description

### Technical field

The present document relates to a method of medical diagnosis, and more particularly to a method of determining the likelihood of a person having a specific disease based on DNA methodology.

### Background

Certain diseases are generally being considered difficult to detect, or diagnostically determine, even with available and verified test methods, such as those where a diagnostic result is compared to a control result. The reason might be that the disease, syndrome or symptoms depend on multiple factors, having very specific interrelations, or that there are no assertive medical diagnostic tests available.

One such disease is bacterial vaginosis (BV), a disease that is characterized by a polymicrobial proliferation of anaerobic bacteria and depletion of lactobacilli in the vagina. Conventionally the diagnosis method used to ascertain if an individual in fact has BV are the Amsel's criteria, where three out of four specific criteria must be fulfilled for a positive BV-diagnosis. The most noticeable drawback is that it requires a clinical examination of the individual and that the assessment is prone to subjectivity, e.g. the whiff test for positive amine. A problem with falsely diagnosing women with BV is that the treatment comprises antibiotics, and that the use thereof should be avoided, on the other hand an untreated BV infection can lead to preterm labor, and other pregnancy related complications, as well as other gynecologic complications.

Several research articles have disclosed that bacteria such as *Gardnerella vaginalis, Mobiluncus curtisii, Mobiluncus mulieris, Bacteroides fragilis, Mycoplasma hominis, Atopbium vaginae, Ureaplasma urealyticum, Megasphera* type I, Clostridia-like bacterial vaginosis associated bacteria (BVABs) 1, 2 and 3, *Sneathia sanguinegens,* and *Mycoplasma genetalium* are considered to be bacterial vaginosis-associated agents (BV-AAs) (Malaguti et al, Biomed res Int. 2015; 2015:645853, Sensitive Detection of Thirteen bacterial Vaginosis-Associated Agents Using Multiplex Polymerase Chain Reaction). Further in US2007/0178495A1 several methods and compositions for identifying these bacterial vaginosis related bacteria are disclosed.

Typical of this disease is that there is no single bacterial strain that characterizes the disease, i.e. it requires a multifactorial analysis. This problem is also associated with many other diseases, syndromes and symptoms connected to different types of bacteria or viruses, or gene mutations or variations.

Other diseases are often characterized by different types markers such as mRNA, miRNA. One disease which could be characterized in this way is for instance cancer, in particular certain tumors, where DNA sampling can be used to predict the risk of developing a certain type of cancer. One such example is described in US5654155A, the hereditary breast cancer diagnostic test, where particular DNA and protein sequences were determined for the BRCA2 gene, which can be used for a diagnostic test to predict the risk of an individual for developing cancer.

EP2507395 discloses a method of detecting the presence or absence of bacteria associated with bacterial vaginosis, and determining a diagnostic score based in the presence or absence of bacteria, and comparing this to a reference score.

There is thus a need for a new method which can reliably predict the likelihood of an individual having or the risk of developing bacterial vaginosis.

### Summary

The invention is defined by the appended independent claims. Embodiments are set forth in the appended dependent claims and in the following description.

There is provided an *in vitro* method of determining the likelihood ratio of an individual having bacterial vaginosis comprising the steps of: performing an analysis on a sample to determine a quantification level value (IQ1, IQ2) of at least two different markers (M1, M2); determining limit quantification values (LT1, LT2) for each of said markers; and determining a likelihood ratio value (LH1, LH2) for each of said marker by comparing the quantification level value (IQ1, Iq2) with the limit quantification values (LT1, LT2); and
calculating the likelihood ratio value of said disease (LHD) through the following algorithm:
LHD = ODDS (disease in general population) *LH1* LH2; wherein when LHD is above 50, or above 70 or above 90 said disease, i.e. according to the invention bacterial vaginosis, is present.

The specific value set for the likelihood ratio of said disease will depend on for instance the normal likelihood of the disease occurring in the general or specific, population, i.e. the ODDS of the disease occurring in general population.

By quantification level value may alternatively also be meant a detection level value, i.e. the detection of the presence or non-presence of a specific marker, according to the invention for bacterial vaginosis.

The limit quantification values are determined based on the validation study results for each marker. In the case of BV analysis, each bacteria (marker) has a limit quantification values which is defined to provide as high sensitivity and specificity as possible.

By determining a likelihood ratio value is meant that the sample results for each marker is assigned a positive likelihood ratio value (LH+/LR+) or negative likelihood ratio value (LH-/LR-), depending on whether the quantification level values, or the normalized quantification values, are below or above a limit quantification values as determined through a validation study.

Through the inventive method there is no need to compare the sample taken from an individual with, e.g. subjective clinical diagnostic methods. The method does not rely on a reference value or score, but is completely based on the calculated likelihood ratio as defined by the algorithm above.

The algorithm could thus also be used for analysis of variables of different genes or gene sequences when determining the risk in multi factorial diseases, such as cancer and Alzheimer's, and in metabolic diseases such as diabetes, however not according to this description.

The first aspect further comprises:
determining a quantification level for a first base marker (IQ1);
normalizing the quantification level values for each of said other species by subtracting the quantification level value (IQ1) of said first base species from said other quantification level values (IQ2); through the formula IQ2-IQ1=ΔIQ2; thereby obtaining a normalized quantification level value (ΔIQ2) for each one of the other species; and wherein the determination of a likelihood ratio value (LH1, LH2) for each of said species is performed by comparing said normalized quantification level values (ΔIQ2) with the limit quantification value (LT1, LT2) for each marker, and
wherein step of the calculating the likelihood ratio value of said disease (LHD) is through the following algorithm: LHD = ODDS (disease in general population)* LH2.

The number of markers used for the determination will depend on the specific disease that is diagnosed. According to the invention, when determining the LHD for bacterial vaginosis 6 different species other than the first species are used. In this case the algorithm would be LHD=ODDS (disease in general population)*LH2*LH3*LH4*LH5*LH6*LH7.

Said analysis may comprise any one of immune assays, polymerase chain reaction (PCR), capillary electrophoresis, Sanger sequencing and next generation sequencing (NGS).

The analysis may further include or comprise other types of *in vitro* analysis methods, depending on the marker to be detected or quantified.

The sample may be any one of a blood sample, a urine sample, a feces sample, a tissue sample, and a swab sample.

The sample used in the *in vitro* method may also be any type of suitable fluid or tissue, and be obtained through any conventional sampling method.

The disease, syndrome or condition could, but not according to this description further be any one of an infection, an inflammation, an immune deficiency, a benign or malign tumor, a genetic disease or syndrome, a neurodegenerative disease or a metabolic disease.

Non-limiting examples of such diseases, syndromes or conditions may be bacterial vaginosis, Alzheimer's or diabetes. The diseases are often multifactorial diseases, syndromes or conditions, such as the bacterial vaginosis, which occurs due to a specific imbalance in the microbiotic flora of the vagina, and requires a multifactorial analysis to be performed to positively determine the correct diagnosis.

According the first aspect the method comprises calculating said limit quantification value (LT1, LT2) from validation study.

### Detailed description

The methods and compositions described herein may be used to detect different types markers, associated with specific diseases, conditions or syndromes, such as infections, cancer, genetic diseases, Parkinson, Alzheimer's disease etc.

Non-limiting examples of markers may be associated with or comprise, for instance bacteria, viruses, tissue, blood, blood components, cells, cell extracts, cell components, stem cells, nerve cells, nucleic acids (DNA), DNA sequences, protein sequences, proteins, enzymes, hormones, neurotransmittors, neurons, antigenes, antibodys, immunoglobulin, etc.. The marker may also be other types of molecules, drugs, metabolites, intermediates, nutrients, substances or agents.

One example of a markers indicative for specific tumors or cancerm such as PSA (prostate specific antigen). In the case of nerodegenrative diseases such as Alzheimer's the markers may include quantification of the level of the neurotransmittor acetylcholine, ACh, in combination with other types of markers. In the example below the markers are associated with the precence of specific bacteria in a vaginal swab sample.

These markers may be either quantified, i.e. a quantification level is detected in the sample, or detected as present or not present in the sample, and weighed in in the calculation of the likelihood ratio for the specific disease, syndrome or condition.

In general, clinical samples, such as swabs, blood samples etc. are taken from individuals, either by their physician or as home samples and are used as the starting material for the analysis.

Common methods for detecting nucleic acids, such as *in vitro* amplification, such as PCR may be used. In case of other markers being used for the *in vitro* detection, methods commonly used in this field are applicable. The clinical samples may be any one of swabs, blood samples, aspirations, tissue sections, biopsies, tissues or fluids obtained and processed through conventional methods.

In the case of detection of a nucleic acid it may be amplified from any type of biological sample, such as a tissue sample, a swab, fluid sample, bacterial organism.

The definitions of certain terms are generally understood to have the meaning as understood by the person skilled in the art.

As used in herein the term "diagnose" is meant as distinguishing or identifying a disease, syndrome or condition, and the term "risk" is meant to indicate an individual's likelihood of having a certain disease, syndrome or condition, or the likelihood of developing a certain disease, syndrome or condition.

In the below example a method for the detection of bacterial vaginosis in a clinical sample from a vaginal swab is disclosed, however this example is not to be considered as limiting the scope of application of the present method and algorithm in respect to diseases, syndromes and conditions that may be identified and/or the risk of developing these.

According to the invention a validation study of bacterial vaginosis is performed to allow for the determination of a likelihood ratio. This study includes the collection of data from individuals and setting a limit value for the presence or absence of a particular marker. The limit value may also be weighted for any specific marker, e.g. in the below example of bacterial vaginosis one bacteria might always be present in the microflora of an individual having BV while another occasionally occurs in the microflora. That means that the bacteria always present affects the total likelihood ratio for BV more than the bacteria which is not always present. In the validation study many different markers, and factors may be evaluated and weighted for a gathered likelihood ratio.

The values from the analysis may be inserted into a database, or a computational program, such as Excel to provide the result.

In one embodiment, an individual performs the sampling at home, for instance as in the example below with a vaginal swab, which swab, or test kit, may for instance be ordered through a webpage, from a net based physician, or bought at a pharmacy or similar store. When the sampling has been performed the individual sends the sample to a clinical diagnostic facility, which performs the *in vitro* analysis as defined in the claims, and sends the result back to the individual by mail, e-mail, through a web-based interface, or to a physician for further decisions on treatment or prescription of drugs.

### Example - bacterial vaginosis

### Material and methods

DNA was extracted from vaginal swabs using ZR-96 Quick-gDNA^{™} (Zymo Research, Irvine, CA, USA). In short, the vaginal swab was lysed in 300µl Genomic Lysis Buffer. 50 µl of the sample was diluted further in 150 µl Genomic Lysis Buffer and disrupted in a TissueLyser (Qiagen, Hilden Germany) for 2 min, 30 Hz, and, of the disrupted sample 100 µl was applied on a Silicon-A^{™} Plate and washed according to the manufacturer's instructions. DNA was eluted in 30 µl Elution buffer.

Primers and TaqMan MGB probes were designed to anneal to ribosomal DNA of *Atopobium vaginae,* Bacterial vaginosis associated bacterium 2 (BVAB2), *Gardnerella vaginalis, Lactobacillus* spp., *Leptotrichia*/*Sneathia* spp., *Megasphaera* spp., and *Mobiluncus* spp. TaqMan probes were labelled with either 6-FAM, VIC or NED as indicated in table 1 to enable multiplex PCR-reactions. Oligonucleotides were from Life Technologies (Foster City, CA, USA). The analysis was made in three reactions; two multiplex reactions, one for *Atopobium vaginae, Gardnerella vaginalis, Leptotrichia*/*Sneathia* spp. and the other for BVAB2, *Megasphaera* spp., *Mobiluncus* spp., and one singleplex reaction *Lactobacillus* spp. Each PCR reaction was performed in PerfeCTa^{®} MultiPlex qPCR SuperMix (Quanta Biosciences, Gaithersbury, MD, USA) with primer and probe concentrations as given in Table 1 to a total volume of 15µl for each reaction.

**Table 1. Primer and probe concentrations used in PCR reactions and fluorophore label of respective probe.**

| Species or genus | Primer concentration (nM) | Probe concentration (nM) | Probe fluorophore |
|---|---|---|---|
| *Atopobium vaginae* | 300 | 100 | VIC |
| BVAB2 | 75 | 100 | VIC |
| *Gardnerella vaginalis* | 600 | 100 | 6-FAM |
| *Lactobacillus* spp. | 75 | 100 | 6-FAM |
| *Leptotrichia*/*Sneathia* spp. | 300 | 100 | NED |
| *Megasphaera* spp. | 75 | 100 | NED |
| *Mobiluncus* spp. | 75 | 100 | 6-FAM |

Sample volume was 3 µl. Analysis was performed on ABI Prism 7300 Sequence Detection System (Life Technologies).

### Data Analysis:

Before further interpretation, Ct values, i.e. quantification level values, from analyses of the bacteria associated with bacterial vaginosis (*Atopobium vaginae, Gardnerella vaginalis, Leptotrichia*/*Sneathia* spp., BVAB2, *Megasphaera* spp., and *Mobiluncus* spp.) were "normalized" by subtracting each Ct value for the specific bacteria with the Ct value from the Lactobacillus spp..

As the Ct value is the logarithm of the bacterial concentration, the resulting ΔCₜ represents the logarithm of the quotient between bacteria and lactobacilli concentrations. For evaluation of the bacterial vaginosis test, the probability of bacterial vaginosis was calculated based on likelihood ratio's (LR) (Fritz and Wainner 2001) for each bacterium associated with bacterial vaginosis. The distribution of individual Ct values for each species was used to manually determine a suitable limit dividing positive LR (LR+) from negative LR (LR-) where a high sensitivity and specificity was obtained.

The LR+ and LR- of each individual bacterium is determined as exemplified below:

| | Positive test result | Negative test result | SUM |
|---|---|---|---|
| Clinical BV positive | 45 (A) | 7 (B) | 52 |
| Clinical BV negative | 26 (C) | 62 (D) | 88 |

The LR+ is calculated as A/(A+B) * (C+D)/C = 45/52 * 88/26 = 2.93 The LR- is calculated as B/(A+B) * (C+D)/D = 7/52 * 88/62 = 0.19 LR+ and LR- for each bacterium was determined as specified in Table 2.

**Table 2 Limits for ΔCt and likelihood ratios for calculation of probability**

| Species or genus | ΔCt limit | LR+ | LR- |
|---|---|---|---|
| *Atopobium vaginae* | 9,7 | 3,67 | 0,07 |
| BVAB2 | 13,5 | 4,82 | 0,29 |
| *Gardnerella vaginalis* | 4,2 | 2,82 | 0,08 |
| *Leptotrichia*/*Sneathia* spp. | 14,1 | 4,06 | 0,70 |
| *Megasphaera* spp. | 14,2 | 3,71 | 0,11 |
| *Mobiluncus* spp. | 16,6 | 3,78 | 0,34 |

When the sample results are analysed each bacterium is assigned the LR+ or LR- value depending on whether the ΔCₜ values are below or above the ΔCₜ limit. If the bacterium cannot be detected in the sample LR- value is assigned.

The LR values are calculated to an ODDS according to the formula: ODDS(BV present) = ODDS(BV in general population) * *LR*(*Atopobium vaginae*) * LR(BVAB2) * *LR*(*Gardnerella vaginalis*) * *LR*(*Leptotrichia*/*Sneathia*) * *LR*(*Megasphaera*) * *LR*(*Mobiluncus*)*.*

The ODDS is further calculated to a probability using the formula probability= ODDS/(ODDS+1). The calculation of probability is exemplified in Table 3. A probability above 70% was considered positive for BV.

**Table 3 Calculation of probability from sample data**

| Sample | ACtA. vaginae | A. vaginae LR | ΔCt BVAB2 | BVAB2 LR | ΔCt G. vaginalis | G. vaginalis LR | ΔCt Leptotric hia/Snea thia | Leptotric hia/Snea thia LR | ΔCt Megasph aera | Megasph aera LR | ΔCt Mobilun cus | Mobilun cus LR | ODDS | Probabili ty (%) |
|---|---|---|---|---|---|---|---|---|---|---|---|---|---|---|
| 1 | 10,53 | 0,07 | | 0,29 | 10,19 | 0,08 | | 0,70 | 6,13 | 3,71 | | 0,34 | 0,00 | 0% |
| 2 | 7,32 | 3,67 | 19,45 | 0,29 | 0,90 | 2,82 | | 0,70 | 3,19 | 3,71 | 22,17 | 0,34 | 0,11 | 10% |
| 3 | | 0,07 | 22,35 | 0,29 | | 0,08 | | 0,70 | 23,40 | 0,11 | 16,41 | 3,78 | 0,00 | 0% |
| 4 | 3,02 | 3,67 | 8,21 | 4,82 | 0,34 | 2,82 | | 0,70 | 3,09 | 3,71 | 23,20 | 0,34 | 58,14 | 98% |
| 5 | | 0,07 | | 0,29 | 15,44 | 0,08 | | 0,70 | | 0,11 | | 0,34 | 0,00 | 0% |
| 6 | | 0,07 | | 0,29 | 15,14 | 0,08 | 14,09 | 4,06 | 23,11 | 0,11 | 19,10 | 0,34 | 0,00 | 0% |
| 7 | -1,80 | 3,67 | -1,17 | 4,82 | -6,75 | 2,82 | | 0,70 | -6,32 | 3,71 | -3,58 | 3,78 | 19,59 | 95% |
| 8 | 2,66 | 3,67 | -2,19 | 4,82 | -2,32 | 2,82 | -3,63 | 4,06 | 7,76 | 3,71 | 4,11 | 3,78 | 3749,11 | 100% |
| 9 | -0,86 | 3,67 | | 0,29 | -1,90 | 2,82 | | 0,70 | -0,33 | 3,71 | 16,53 | 3,78 | 38,89 | 97% |
| 10 | 4,97 | 3,67 | | 0,29 | 2,85 | 2,82 | | 0,70 | 7,45 | 3,71 | | 0,34 | 3,50 | 78% |

## Claims

1. An *in vitro* method of determining the likelihood ratio of an individual having, or obtaining, bacterial vaginosis comprising the steps of:
performing an analysis on a sample to determine a quantification level value (IQ1, IQ2) of at least two different species (M1, M2) for bacterial vaginosis;
determining limit quantification values (LT1, LT2) for each of said species, wherein said limit quantification value (LT1, LT2) are calculated from a validation study and determining a likelihood ratio value (LH1, LH2) for each of said species by comparing the quantification level value (IQ1, IQ2) with the limit quantification values (LT1, LT2); wherein the method further comprises:
determining a quantification level for a first base species (IQ1);
normalizing the quantification level values for each of said other species by subtracting the quantification level value (IQ1) of said first base species from said other quantification level values (IQ2); through the formula IQ2-IQ1=ΔIQ2;
thereby obtaining a normalized quantification level value (ΔIQ2) for each one of the other species; and
wherein the determination of a likelihood ratio value (LH1, LH2) for each of said species is performed by comparing said normalized quantification level values (ΔIQ2) with the limit quantification value (LT1, LT2) for each species,
and wherein the step of the calculating the likelihood ratio value of bacterial vaginosis (LHD) is through the following algorithm, wherein six different species other than the first base species are used:
LHD = ODDS (disease in general population)* LH2*LH3*LH4*LH5*LH6*LH7.

2. The *in vitro* method as claimed in claim 1, wherein
when the likelihood ratio for the disease (LHD) is above 50, or above 70 or above 90 said bacterial vaginosis is present.

3. The *in vitro* method of claim 1, wherein said analysis comprises any one of immune assays, polymerase chain reaction (PCR), capillary electrophoresis, Sanger sequencing and next generation sequencing (NGS).

4. The *in vitro* method as claimed in claim 1 or 2, wherein said sample is

## Patentansprüche

1. *In vitro*-Verfahren zur Bestimmung des Likelihood-Verhältnisses eines Individuums, bakterielle Vaginose zu haben oder zu bekommen, umfassend die Schritte:
Durchführen einer Analyse an einer Probe, um einen Quantifizierungsniveauwert (IQ1, IQ2) von mindestens zwei unterschiedlichen Spezies (M1, M2) für bakterielle Vaginose zu bestimmen;
Bestimmen von Grenzquantifizierungswerten (LT1, LT2) für jede der Spezies, wobei die Grenzquantifizierungswerte (LT1, LT2) aus einer Validierungssstudie berechnet werden, und
Bestimmen eines Likelihood-Verhältniswertes (LH1, LH2) für jede der Spezies durch Vergleichen des Quantifizierungsniveauwertes (IQ1, IQ2) mit den Grenzquantifizierungswerten (LT1, LT2); wobei das Verfahren ferner umfasst:
Bestimmen eines Quantifizierungsniveaus für eine erste Basenspezies (IQ1);
Normalisieren der Quantifizierungsniveauwerte für jede der anderen Spezies durch Subtrahieren des Quantifizierungsniveauwertes (IQ1) der ersten Basenspezies von den anderen Quantifizierungsniveauwerten (IQ2); durch die Formel IQ2-IQ1 = ΔIQ2;
dadurch Erhalten eines normalisierten Quantifizierungsniveauwertes (ΔIQ2) für jede der anderen Spezies; und
wobei die Bestimmung eines Likelihood-Verhältniswertes (LH1, LH2) für jede der Spezies durch Vergleichen der normalisierten Quantifizierungsniveauwerte (ΔIQ2) mit dem Grenzquantifizierungswert (LT1, LT2) für jede Spezies durchgeführt wird, und
wobei der Schritt des Berechnens des Likelihood-Verhältniswertes der bakteriellen Vaginose (LHD) durch den folgenden Algorithmus erfolgt, wobei sechs unterschiedliche Spezies verwendet werden, die anders als die erste Basenspezies sind:
LHD = ODDS (Erkrankung in der allgemeinen Population)* LH2^{∗}LH3^{∗}LH4^{∗}LH5^{∗}LH6^{∗}LH7.

2. *In vitro*-Verfahren wie in Anspruch 1 beansprucht, wobei die bakterielle Vaginose vorliegt, wenn das Likelihood-Verhältnis für die Erkrankung (LHD) über 50 oder über 70 oder über 90 liegt.

3. *In vitro*-Verfahren nach Anspruch 1, wobei die Analyse einen aus Immunassays, Polymerasekettenreaktion (PCR), Kapillarelektrophorese, Sanger-Sequenzierung und Next-Generation-Sequencing (NGS) umfasst.

4. *In vitro*-Verfahren wie in Anspruch 1 oder 2 beansprucht, wobei die Probe eine Tupferprobe ist.

## Revendications

1. Procédé in vitro de détermination du rapport de probabilité d'un individu à avoir, ou à obtenir, une vaginose bactérienne comprenant les étapes de :
réalisation d'une analyse sur un échantillon pour déterminer une valeur de niveau de quantification (IQ1, IQ2) d'au moins deux espèces différentes (M1, M2) pour la vaginose bactérienne ;
détermination de valeurs de quantification limites (LT1, LT2) pour chacune desdites espèces, dans lequel lesdites valeurs de quantification limites (LT1, LT2) sont calculées à partir d'une étude de validation ; et
détermination d'une valeur de rapport de probabilité (LH1, LH2) pour chacune desdites espèces par comparaison de la valeur de niveau de quantification (IQ1, IQ2) aux valeurs de quantification limites (LT1, LT2) ; dans lequel le procédé comprend en outre :
la détermination d'un niveau de quantification pour une première espèce de base (IQ1) ;
la normalisation des valeurs de niveau de quantification pour chacune desdites autres espèces par soustraction de la valeur de niveau de quantification (IQ1) de ladite première espèce de base auxdites autres valeurs de niveau de quantification (IQ2) ; par l'intermédiaire de la formule IQ2 - IQ1 = ΔIQ2 ;
permettant ainsi d'obtenir une valeur de niveau de quantification normalisée (ΔIQ2) pour chacune des autres espèces ; et
dans lequel la détermination d'une valeur de rapport de probabilité (LH1, LH2) pour chacune desdites espèces est réalisée par comparaison desdites valeurs de niveau de quantification normalisées (ΔIQ2) à la valeur de quantification limite (LT1, LT2) pour chaque espèce,
et dans lequel l'étape de calcul de la valeur de rapport de probabilité d'une vaginose bactérienne (LHD) est par l'intermédiaire de l'algorithme suivant, dans lequel six espèces différentes autres que la première espèce de base sont utilisées :
LHD = ODDS (maladie dans la population générale)* LH2*LH3*LH4*LH5*LH6*LH7.

2. Procédé in vitro selon la revendication 1, dans lequel
lorsque le rapport de probabilité pour la maladie (LHD) est supérieur à 50, ou supérieur à 70 ou supérieur à 90 ladite vaginose est présente.

3. Procédé in vitro selon la revendication 1, dans lequel ladite analyse comprend l'un quelconque de dosages immunologiques, d'une réaction de polymérisation en chaîne (PCR), d'une électrophorèse capillaire, d'un séquençage de Sanger et d'un séquençage de nouvelle génération (NGS).

4. Procédé in vitro selon la revendication 1 ou 2, dans lequel ledit échantillon est un frottis.
